(19) Europäisches Patentamt
European Patent Office
Office européen des brevets

(11) EP 3 826 985 B1

(12) **FASCICULE DE BREVET EUROPEEN**

(45) Date de publication et mention de la délivrance du brevet:
**04.05.2022 Bulletin 2022/18**

(21) Numéro de dépôt: **19742241.3**

(22) Date de dépôt: **25.07.2019**

(51) Classification Internationale des Brevets (IPC):
***C07C 43/11*** ^(2006.01) ***C02F 1/26*** ^(2006.01)
***C02F 1/68*** ^(2006.01) ***C08G 83/00*** ^(2006.01)

(52) Classification Coopérative des Brevets (CPC):
**C02F 1/26; C02F 1/683; C07C 43/11;** C02F 1/444; C02F 2101/103; C02F 2101/20; C02F 2101/22; C02F 2101/30; C02F 2101/305; C02F 2101/306; C02F 2103/007; C02F 2103/06; C02F 2103/10; C08G 83/003

(86) Numéro de dépôt international:
**PCT/EP2019/070097**

(87) Numéro de publication internationale:
**WO 2020/021034 (30.01.2020 Gazette 2020/05)**

(54) **COMPOSES DE TYPE DENDRIMERE, LEURS PROCEDES DE PREPARATION ET LEURS UTILISATIONS**

DENDRIMERVERBINDUNGEN, VERFAHREN ZU IHRER HERSTELLUNG UND IHRE VERWENDUNGEN

DENDRIMER-TYPE COMPOUNDS, METHODS FOR PRODUCING SAME, AND USES THEREOF

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **26.07.2018 FR 1856990**

(43) Date de publication de la demande:
**02.06.2021 Bulletin 2021/22**

(73) Titulaire: **Université De Reims Champagne-Ardenne
51100 Reims (FR)**

(72) Inventeurs:
- **BOUQUILLON, Sandrine
51100 Reims (FR)**
- **HAYOUNI, Safa
59000 Lille (FR)**
- **MENOT, Bérangère
51100 Reims (FR)**

(74) Mandataire: **Cabinet Becker et Associés
25, rue Louis le Grand
75002 Paris (FR)**

(56) Documents cités:
**US-A1- 2002 035 238**

- **BÉRENGÉRE MENOT ET AL: "Synthesis of surface-modified PAMAMs and PPIs for encapsulation purposes: influence of the decoration on their sizes and toxicity", TETRAHEDRON, vol. 71, no. 21, 1 mai 2015 (2015-05-01), pages 3439-3446, XP055568409, AMSTERDAM, NL ISSN: 0040-4020, DOI: 10.1016/j.tet.2015.03.078 cité dans la demande**
- **CHUNG-JUI SU ET AL: "Extraction of Cupric Ions with Ionic Liquids Containing Polypyridine-type Small Molecules or Peripherally Pyridine-modified Dendrimers", CHEMISTRY - AN ASIAN JOURNAL, vol. 7, no. 10, 1 octobre 2012 (2012-10-01), pages 2438-2445, XP055594144, DE ISSN: 1861-4728, DOI: 10.1002/asia.201200447**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 3 826 985 B1

## Description

OBJET DE L'INVENTION

La présente invention concerne le domaine des dendrimères, notamment des composés dendrimères et leur utilisation dans le piégeage de composés chimiques, de préférence des polluants.

ARRIERE-PLAN DE L'INVENTION

Depuis plusieurs années, la recherche s'intéresse à la synthèse de nouvelles molécules de type « dendrimère ». Tel un polymère, un dendrimère est une macromolécule issue de la répétition d'une même unité, et dont l'architecture n'est pas linéaire mais organisée en branches pouvant s'apparenter à celles d'un arbre (« dendron » = arbre en grec). De par leur forme sphérique et la présence de « cavités » internes, ces dendrimères offrent la possibilité de piéger divers composés chimiques, tels que des métaux ou des composés organiques. De nombreuses applications peuvent découler de cette propriété, en particulier dans le domaine de la dépollution de l'eau, l'extraction d'éléments chimiques issus de minerais ou encore l'encapsulation de substances actives.

En raison des contraintes environnementales engendrées par une utilisation excessive des ressources fossiles (épuisement des ressources naturelles, gaz à effet de serre), l'introduction progressive des matières issues de la biomasse dans des processus courants de la chimie devient un enjeu important dans notre société. C'est pourquoi, le développement de dendrimères utilisant des dérivés biosourcés, en particulier les hydrates de carbone tels que le pentose ou encore le glycérol, capables de piéger des composés chimiques a suscité une attention particulière au cours de ces dernières années. En particulier, les dendrimères résultant de la fonctionnalisation périphérique de dendrimères commerciaux par des entités hydrophiles issues d'agro-ressources ont révélé de nombreux avantages par rapport à leurs précurseurs commerciaux, telles qu'une meilleure tolérance physiologique, une moindre cytotoxicité et écotoxicité et une excellente hydrosolubilité.

Dans ce contexte, Balieu et al. (Adv. Synth. Catal. 2010, 352, 1826-1833) ont décrit la synthèse de dendrimères de type poly(propyleneimines) (PPI) modifiés en périphérie par des motifs glycéryles, en utilisant le glycérol comme précurseur. Balieu et al. ont également décrit l'utilisation de tels dendrimères GD-PPI (« glycerol-decorated-PPI ») pour encapsuler des agents de contraste (J. Biomed. Mater. Res. A. 2013, 101A, 613-621).

Menot et al. (Tetrahedron, 2015, 71, 3439-3446) ont décrit la synthèse de dendrimères de type poly(amidoamine) (PAMAM) également modifiés en périphérie par des motifs glycéryles GD-PAMAM (« glycerol-decorated-PAMAM »), et ont étudié la capacité de ces dendrimères à piéger des composés organiques, tels que le diclofénac, le β-estradiol, l'atrazine et le diuron.

De ces études, il en résulte que les dendrimères GD-PAMAM et GD-PPI sont moins cytotoxiques que les dendrimères précurseurs commerciaux respectifs PAMAM et PPI. Toutefois, ils présentent une capacité de piégeage plus limitée.

Egalement, des dendrimères synthétisés entièrement à partir de dérivés biosourcés de type polyéther-polyol ont été utilisés pour leur rôle de tensioactif ou de stabilisateur. Par exemple, CA 2,356,474 décrit la synthèse de dendrimères de type polyéther-polyol préparés à partir du glycérol, et leurs utilisations comme agents stabilisateur dans des compositions. CN 102389746 décrit la synthèse d'un dendrimère de type polyéther-polyol de première génération également préparé à partir du glycérol et son utilisation comme agent tensioactif.

Il subsiste donc un réel besoin de développer des dendrimères à partir de ressources renouvelables, qui combinent à la fois de bonnes propriétés de piégeage ou d'encapsulation de composés chimiques, notamment des polluants, et une faible cytotoxicité et écotoxicité.

RESUME DE L'INVENTION

Devant cette problématique, les inventeurs ont démontré de manière surprenante que des composés de type dendrimère de génération (n), de type polyéther-polyol, possédaient d'excellentes capacités de piégeage, notamment vis-à-vis des métaux et des composés organiques. Ces composés de type dendrimère de génération (n) répondent également aux contraintes environnementales du fait de leur faible cytotoxicité et écotoxicité. Les inventeurs ont en outre démontré le caractère réversible du piégeage de composés chimiques par ces composés de type dendrimère de génération (n), offrant ainsi la possibilité de recycler le dendrimère.

La présente invention concerne donc l'utilisation d'au moins un composé de type dendrimère de génération (n) ou un de ses sels comprenant :

- un motif central de valence 3 représenté par la formule (I) :

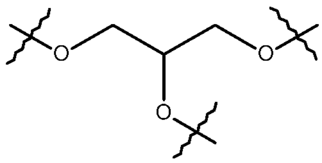

(I) ;

- (n-1) couches internes de génération composées d'unités de répétitions disposées en arborescence autour du motif central et représentées par la formule (II) :

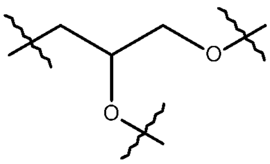

(II) ;

- une couche externe composée d'unités représentées par la formule III :

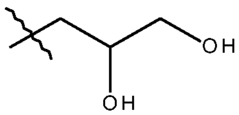

(III) ;

lesdites unités de formule (III) étant liées audit motif central quand n est 1 ou à la couche interne la plus éloignée dudit motif central quand n est différent de 1 ; et
dans lequel n est un entier compris entre 1 et 12,
pour le piégeage d'au moins un composé chimique, de préférence un polluant, contenu dans un échantillon liquide.

Selon un mode particulier de l'invention, ladite couche externe comprend $3 \times 2^{(n-1)}$ unités de formule (III).
Selon un autre mode particulier de l'invention, n est un entier compris entre 1 et 10, de préférence entre 1 et 5, et de manière encore plus préférée n est 1, 2, ou 3.
Selon un mode préféré, le composé de type dendrimère de génération (n) utilisé dans la présente invention est représenté par l'une des structures suivantes :

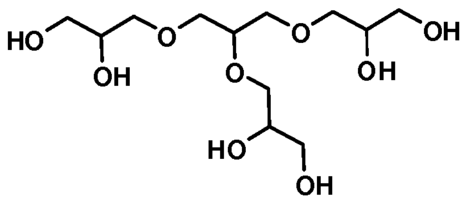

II.2,

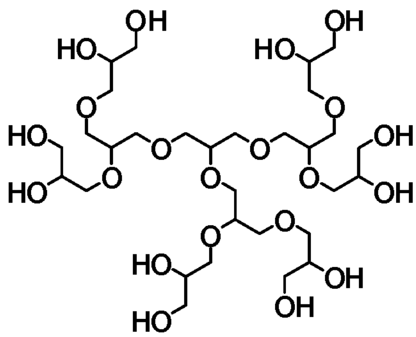

II.4, et

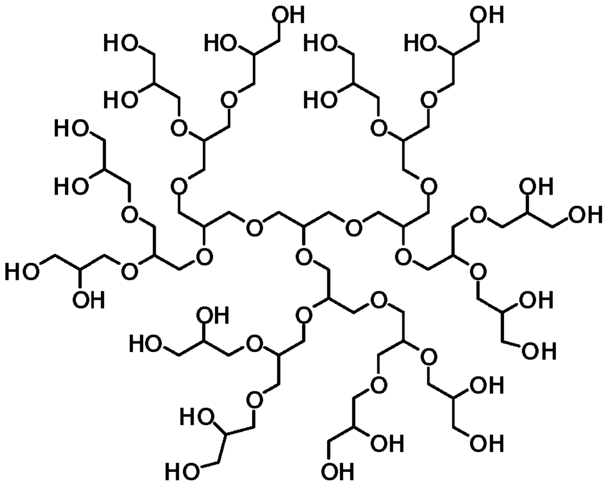

II.6.

L'invention concerne également un procédé de piégeage d'au moins un composé chimique, de préférence un polluant, contenu dans un échantillon liquide, comprenant les étapes suivantes :

(a) la mise en contact dudit échantillon avec au moins un composé de type dendrimère de génération (n) tel que défini dans la présente demande ;
(b) optionnellement, l'extraction dudit au moins un composé de type dendrimère de génération (n) piégeant ledit au moins un composé chimique, de préférence par dialyse ou ultrafiltration ;
(c) optionnellement, la libération dudit au moins un composé chimique par mise en contact d'un acide avec ledit au moins un composé de type dendrimère de génération (n) piégeant ledit au moins un composé chimique ; et
(d) optionnellement, la séparation dudit au moins un composé de type dendrimère de génération (n) et dudit au moins un composé chimique, de préférence, par dialyse ou ultrafiltration.

Selon un mode préféré de l'invention, ledit au moins un composé de type dendrimère de génération (n) obtenu à l'étape (d) est remis en œuvre à l'étape (a).

Selon un mode particulier de l'invention, ledit polluant est un métal, de préférence choisi parmi le mercure, le chrome, le cérium, le plomb, le cadmium, le cuivre, l'arsenic, le nickel, le zinc, le cobalt, le manganèse, l'antimoine, l'étain, et l'argent.

Selon un autre mode particulier de l'invention, ledit polluant est un composé organique d'origine naturelle ou synthétique, de préférence choisi parmi le diclofénac, le glyphosate, l'atrazine, l'estradiol, le diuron, ou un de leurs sels et/ou un de leurs dérivés.

L'invention concerne aussi l'utilisation d'un composé de type dendrimère de génération (n) tel que défini dans la présente demande, ou le procédé de piégeage tel que défini dans la présente demande, pour la dépollution d'un effluent ménager, hospitalier, industriel, ou minier, et/ou pour la dépollution d'un cours d'eau et/ou d'une nappe phréatique, et/ou pour l'extraction de composés chimiques, tels que des métaux, en particulier les terres rares.

Un autre objet concerne un composé de type dendrimère de génération (n) ou un de ses sels comprenant :

- un motif central de valence 3 représenté par la formule (I) :

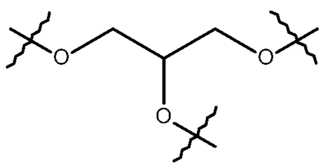

(I) ;

- (n-1) couches internes de génération composées d'unités de répétitions disposées en arborescence autour du motif central et représentées par la formule (II) :

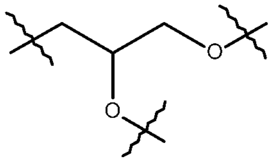

(II) ;

- une couche externe composée d'unités représentées par la formule (III) :

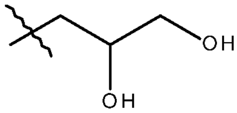

(III),

lesdites unités de formule (III) étant liées à la couche interne la plus périphérique (éloignée) dudit motif central ; et dans lequel n est compris entre 3 et 12.

Selon un mode préféré, le composé de type dendrimère de génération (n) est représenté par la structure suivante :

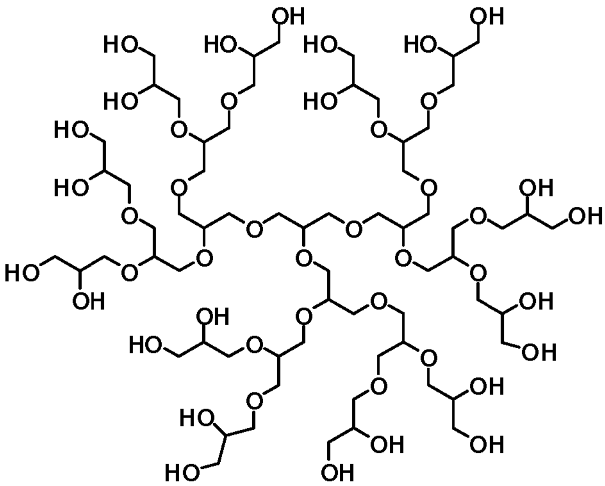

II.6.

BREVE DESCRIPTION DES FIGURES

**Figure 1** : Schéma illustrant la dépollution d'un cours d'eau mettant en œuvre un composé de type dendrimère de génération (n) selon l'invention.
**Figure 2** : Schéma illustrant le procédé de piégeage réversible d'un polluant par un composé de type dendrimère de génération (n) selon l'invention.

DESCRIPTION DETAILLEE

La présente invention porte sur l'utilisation d'au moins un composé de type dendrimère de génération (n) de type polyéther-polyol synthétisé à partir du glycérol, pour le piégeage d'au moins un composé chimique, de préférence un polluant, contenu dans un échantillon liquide. Par ailleurs, ce composé de type dendrimère de génération (n) est parfaitement adapté aux contraintes environnementales en présentant une faible cytotoxicité et écotoxicité. Ce composé de type dendrimère de génération (n) peut également être recyclé pour la mise en œuvre du procédé de piégeage.

Composés

Les composés de type dendrimère de génération (n) tels que définis dans la présente demande et utilisés pour

le piégeage d'au moins un composé chimique, de préférence un polluant, sont des dendrimères de type polyéther-polyol et sont construits à partir d'un motif central glycérol. Plus spécifiquement, les composés de type dendrimère de génération (n), ou leurs sels, utilisés selon l'invention pour le piégeage d'au moins un composé chimique, de préférence un polluant, contenu dans un échantillon liquide comprennent :

- un motif central de valence 3 représenté par la formule (I) :

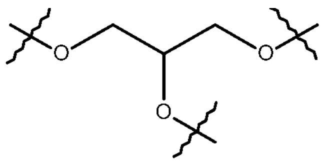

(I) ;

- (n-1) couches internes de génération composées d'unités de répétitions disposées en arborescence autour du motif central et représentées par la formule (II) :

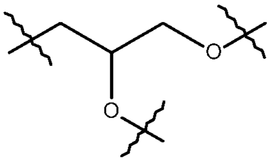

(II) ;

- une couche externe composée d'unités représentées par la formule (III) :

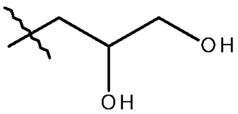

(III) ;

lesdites unités de formule (III) étant liées audit motif central quand n est 1 ou à la couche interne la plus éloignée dudit motif central quand n est différent de 1; et dans lequel n est un entier compris entre 1 et 12.

Par sel, on entend des sels de groupes acides ou basiques du composé de type dendrimère de génération (n) spécifié. Les sels d'acide comprennent, mais ne sont pas limités à des sels de chlorhydrate, bromhydrate, iodhydrate, nitrate, sulfate, bisulfate, phosphate, phosphate acide, isonicotinate, acétate, lactate, salicylate, citrate, tartrate, pantothénate, bitartrate, ascorbate, succinate, maléate, gentisinate, fumarate, gluconate, glucaronate, saccharate, formate, benzoate, glutamate, méthanesulfonate, éthanesulfonate, benzènesulfonate, et paratoluènesulfonate. Des sels de base comprennent, mais ne sont pas limités à des sels d'aluminium, calcium, lithium, magnésium, potassium, sodium, zinc, et diéthanolamine.

Selon l'invention, le motif central du composé de type dendrimère de génération (n) représenté par la formule (I) est un dérivé biosourcé correspondant au glycérol. La valence du glycérol correspond au nombre de bras qu'il possède. Il peut ainsi former un nombre de trois liaisons et être donc lié à trois unités de répétitions de formule (II) lorsque n est différent de 1 ou à trois unités de formule (III) lorsque n est 1. De préférence, le motif central est lié à trois unités de répétitions de formule (II) lorsque n est différent de 1 ou à trois unités de formule (III) lorsque n est 1.

Les (n-1) couches internes de génération du composé de type dendrimère de génération (n) sont composées d'unités de répétitions représentées par la formule (II) et sont disposées en arborescence autour du motif central tel que défini ci-dessus. On entend par « en arborescence », une disposition selon une structure qui s'apparente aux branches d'un arbre. Ces (n-1) couches sont dites « internes » car elles sont comprises entre le motif central et la couche externe composée d'unités représentées par la formule (III). Les unités de répétition de formule (II) composant une couche interne de génération sont ainsi reliées aux unités de répétition de formule (II) de la couche de génération inférieure ou audit motif central de formule (I), et aux unités de formule (II) de la couche de génération supérieure ou aux unités de formule (III) de la couche externe.

La couche externe du composé de type dendrimère de génération (n) est composée d'unités représentées par la formule (III). Cette couche est dite « externe » car elle correspond à la couche la plus éloignée du motif central. Les unités de formule (III) composant la couche externe sont ainsi reliées aux unités de formule (II) de la couche interne de génération inférieure lorsque n est différent de 1, ou reliées au motif central de formule (I) lorsque n est 1.

Dans le cadre de la présente invention

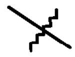

représente le point d'attache, i.e. la liaison covalente, entre le motif central, les unités de répétitions de formule (II) des (n-1) couches internes de génération, et les unités de formule (III) de la couche externe. De manière préférentielle, cette liaison covalente est une liaison carbone-oxygène.

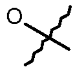

représente un atome d'oxygène relié au point d'attache tel que défini ci-dessus. Selon un mode particulier,

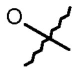

représente un groupement hydroxyle OH.

Un composé de type dendrimère selon l'invention est dit « de génération n » ou « de n$^{ième}$ génération » lorsqu'il possède n couches de génération organisées autour du motif central. n est ainsi un nombre entier correspondant au nombre de générations du composé de type dendrimère selon l'invention.

Selon un mode particulier de l'invention, n est compris entre 1 et 10, de manière plus préférée entre 1 et 5, et de manière encore plus préférée n est 1, 2, ou 3.

Selon le mode de réalisation particulier dans lequel n est égal à 1, un composé de type dendrimère de génération 1 comprend un motif central de valence 3 de formule (I) et une couche externe composée d'unités de formule (III). Selon ce mode de réalisation, le composé de type dendrimère de génération 1 ne comprend pas de couche interne de génération. De préférence, il répond à la formule suivante :

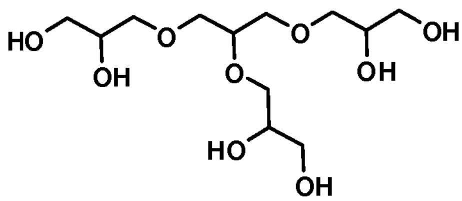

II.2.

Selon le mode de réalisation particulier dans lequel n est égal à 2, un composé de type dendrimère de génération 2 comprend un motif central de valence 3 de formule (I), une couche interne composée d'unités de répétition de formule (II), et une couche externe composée d'unités de formule (III). Selon ce mode de réalisation, le composé de type dendrimère de génération (2) comprend une seule couche interne. De préférence, il répond à la formule suivante :

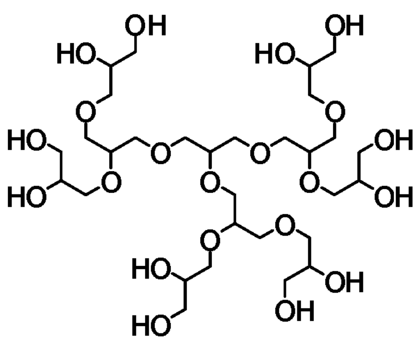

II.4.

Selon le mode de réalisation particulier dans lequel n est égal à 3, un composé de type dendrimère de génération 3 comprend un motif central de valence 3 de formule (I), deux couches internes composées d'unités de répétition de formule (II), et une couche externe composée d'unités de formule (III). Selon ce mode de réalisation, le composé de

type dendrimère de génération (3) comprend deux couches internes. De préférence, il répond à la formule suivante :

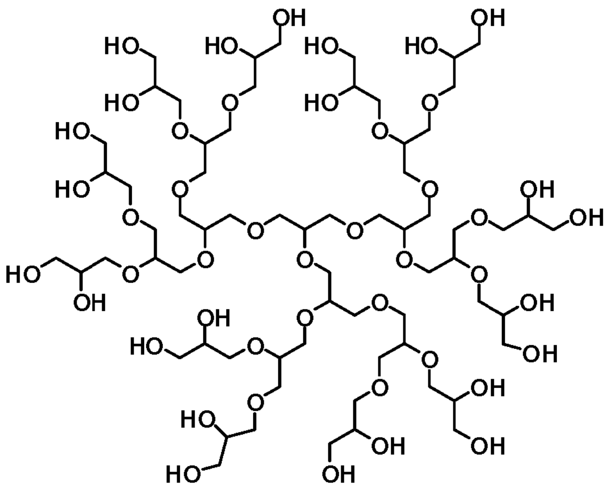

II.6.

Selon le mode particulier dans lequel n est compris entre 4 et 12, c'est-à-dire n est 4, 5, 6, 7, 8, 9, 10, 11 ou 12, un composé de type dendrimère de génération n ou de $n^{ième}$ génération comprend un motif de valence 3 de formule (I), (n-1) couches internes de génération composées d'unités de répétition de formule (II), et une couche externe composée d'unités de formule (III).

Selon un mode préféré de l'invention, la couche externe du composé de type dendrimère de génération (n) comprend $3 \times 2^{(n-1)}$ unités de formule (III), où n est tel que défini ci-dessus, de sorte qu'une arborescence totale est obtenue.

Un composé de type dendrimère de génération (n) présentant une « arborescence totale » désigne un composé de type dendrimère possédant le nombre maximal d'unités de formule (III) pouvant composer ladite couche externe, à savoir $3 \times 2^{(n-1)}$ unités. Ce nombre maximal est atteint lorsque le nombre maximal d'unités de répétitions de formule (II) est atteint pour chacune des (n-1) couches internes et lorsque le nombre maximal d'unités de formule (III) de la couche externe est également atteint.

Plus particulièrement, le nombre N total d'unités d'un composé de type dendrimère de génération (n) présentant une arborescence totale est défini par la formule suivante :

$$N = \sum_{k=1}^{n} 3 \times 2^{(k-1)}$$

Un composé de type dendrimère de génération (n) présente ainsi une « arborescence totale » lorsque toutes les fonctions réactives, à savoir les hydroxyles, des composés permettant d'obtenir les unités de répétition de formule (II) des (n-1) couches internes de génération et toutes les fonctions réactives du motif central de formule (I) (i.e. les hydroxyles du glycérol) sont substituées par les unités de répétition de formule (II) de la couche interne supérieure et par les unités de répétition de formule (III) de la couche externe. Par opposition à une arborescence totale, un composé de type dendrimère de génération présente une « arborescence partielle » lorsqu'au moins une fonction réactive (i.e. au moins un hydroxyle) d'au moins un composé permettant d'obtenir une unité de répétition de formule (II) d'au moins une couche interne de génération ou au moins une fonction réactive du motif central de formule (I) (i.e. au moins un hydroxyle du glycérol) n'est pas substituée se retrouvant ainsi sous la forme -OH. Dans ce cadre d'une arborescence partielle,

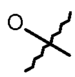

des formules (I) et (II) peut représenter un groupement hydroxyle OH.

Dans le cadre d'une arborescence totale et lorsque n est 1, un composé de type dendrimère de génération 1 comprend un motif central de valence 3 de formule (I) et une couche externe composée de 3 unités de formule (III) disposées en arborescence autour du motif central. Dans le cadre d'une arborescence totale et lorsque n est 2, un

composé de type dendrimère de génération 2 comprend un motif central de valence 3 de formule (I), une couche interne de génération composées de 3 unités de répétitions de formule (II) disposées en arborescence autour du motif central, et une couche externe composée de 6 unités de formule (III) disposées en arborescence autour de la couche interne.

Dans le cadre d'une arborescence totale et lorsque n est 3, un composé de type dendrimère de génération 3 comprend un motif central de valence 3 de formule (I), une première couche interne de génération composées de 3 unités de répétitions de formule (II) disposées en arborescence autour du motif central, une seconde couche interne de génération composées de 6 unités de répétitions de formule (II) disposées en arborescence autour de la première couche interne, et une couche externe composée de 12 unités de formule (III) disposées en arborescence autour de la seconde couche interne.

Un autre objet de l'invention concerne aussi un composé de type dendrimère de génération (n), ou un de ses sels, tel que défini ci-dessus, avec n compris entre 3 et 12. Plus particulièrement, l'invention porte sur un composé de type dendrimère de génération (n) ou un de ses sels comprenant :

- un motif central de valence 3 représenté par la formule (I) :

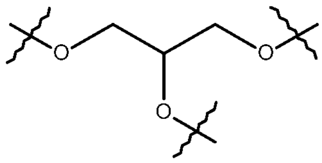

(I) ;

- (n-1) couches internes de génération composées d'unités de répétitions disposées en arborescence autour du motif central et représentées par la formule (II) :

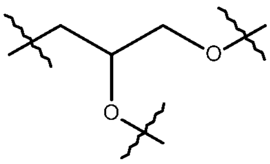

(II) ;

- une couche externe composée d'unités représentées par la formule (III) :

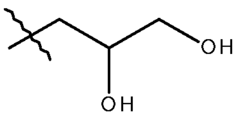

(III),

lesdites unités de formule (III) étant liées à la couche interne la plus périphérique (éloignée) dudit motif central ; et dans lequel n est un entier compris entre 3 et 12.

Selon un mode préféré, le composé de type dendrimère de génération (n) répond à la formule suivante :

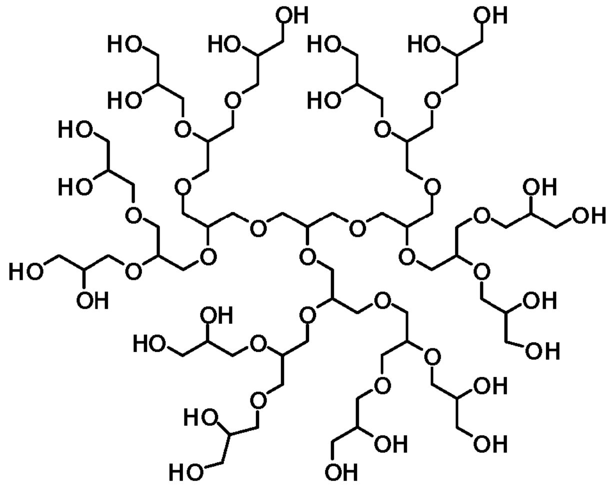

II.6.

Procédé

Les composés de type dendrimère de génération (n) tels que défini dans la présente demande sont, par exemple, préparés à partir d'un procédé comprenant : a) une étape d'initiation, b) une étape optionnelle de propagation mise en œuvre (n-1) fois, et c) une étape de terminaison, dans laquelle n est compris entre 1 et 12. On comprend donc que le procédé de préparation d'un composé de type dendrimère de génération (1) comprend uniquement une étape d'initiation et une étape de terminaison.

De préférence, les différentes couches de génération sont obtenues en générations successives en partant du motif central, selon un procédé tel que celui décrit plus loin. Les dendrimères obtenus à chaque génération, c'est-à-dire ayant un nombre déterminé de couches, peuvent être isolés. Un composé de type dendrimère de génération (n) selon l'invention peut être obtenu à partir d'un composé de type dendrimère de génération (n-1). Chaque intermédiaire obtenu à la fin de chaque étape ou sous-étape peut être isolé, et éventuellement purifié, et mis en réaction dans l'étape suivante. Plusieurs étapes ou sous-étapes du procédé peuvent être réalisées successivement sans isoler d'intermédiaire.

Les différentes étapes a)-c) citées précédemment peuvent être décomposées en plusieurs étapes, éventuellement nommées « sous-étapes ».

L'étape (a) du procédé comprend une réaction d'allylation des groupes hydroxyles du glycérol. Dans un mode de réalisation particulier, le glycérol est mis en contact avec une source d'allylation telle que le bromure d'allyle ou un sulfonate d'allyle et de préférence une base, telle que l'hydroxyde de sodium. La réaction peut être réalisée à une température comprise entre - 10 °C et 40°C, de préférence entre -5 et 10 °C. La réaction peut être réalisée pendant au moins 12 heures, de manière préférée au moins 24 heures et de manière encore plus préférée au moins 48 heures. La réaction est préférablement réalisée dans un solvant organique. Des exemples de solvants organiques non limitatifs sont le méthanol, l'éthanol, l'acétone, le cyclohexane, le benzène, le toluène, l'acétonitrile, le DMF, le DMSO, le diéthyléther, l'acétate d'éthyle, le tétrahydrofurane, le dichlorométhane, et leurs mélanges. De préférence, le solvant organique est le DMF. Le nombre d'équivalents de la source d'allylation par fonction hydroxyle, i.e. 3 pour le glycérol, est avantageusement compris entre 1 et 2,5 équivalents, de préférence entre 1,1 et 1,5 équivalents. Le nombre d'équivalents de la base par fonction hydroxyle est avantageusement compris entre 1 et 4 équivalents, de préférence entre 2 et 3 équivalents.

Le composé obtenu à l'étape a) décrite ci-dessus, ou à l'étape b-2) décrite ci-dessous, est optionnellement soumis à une étape de propagation (b). L'étape (b) comprend notamment deux sous-étapes successives.

La sous-étape b-1) comprend une réaction de dihydroxylation des doubles liaisons carbone-carbone des motifs allyles. Dans un mode particulier, le réactif permettant la réaction de dihydroxylation est le couple AD-mix-β/$Na_2SO_3$. Dans ce mode, le ratio AD-mix-β/$Na_2SO_3$ est de préférence compris entre 0.8:1 et 1:0.8 (w/w). La réaction peut être réalisée dans un solvant ou mélange de solvants, tel qu'un mélange tBuOH/$H_2O$. La température de la réaction est avantageusement comprise entre -10 et 40 °C, de manière plus préférée entre 0 et 25 °C.

La sous-étape b-2) comprend une réaction d'allylation des groupes hydroxyles du composé obtenu à la sous-étape b-1). Les conditions utilisées pour la mise en œuvre de cette sous-étape b-2) sont de préférence similaires à celles décrites ci-dessus pour l'étape (a).

L'étape b) de propagation comprenant les sous-étapes b-1) et b-2), est réalisée (n-1) fois, de manière cyclique, en vue de l'obtention d'un composé de type dendrimère de génération (n) tel que décrit ci-dessus.

Par exemple, pour un composé de type dendrimère de génération (2), le cycle b-1) - b-2) est réalisé une fois. Par exemple, pour un composé de type dendrimère de génération (3), le cycle b-1) - b-2) est réalisé deux fois.

Il est bien entendu que le composé de type dendrimère de génération (n) n'est finalement obtenu qu'à l'issue de l'étape de terminaison (c), qui suit l'étape b) réalisée (n-1) fois. On comprend donc que, pour un composé de type dendrimère de génération 1 tel que décrit ci-dessus, l'étape (b) n'est pas réalisée et l'étape (a) est suivie de l'étape (c).

Le composé obtenu à l'étape a) lorsque n est 1 ou obtenu à l'étape b-2) lorsque n est compris entre 2 et 12, est soumis à une étape de terminaison (c).

L'étape c) comprend une réaction de dihydroxylation de la double liaison carbone-carbone des motifs allyles du composé obtenu à l'étape a) ou obtenu à l'étape b-2). Les conditions utilisées pour la mise en œuvre de cette étape c) sont de préférence similaires à celles décrites ci-dessus pour la sous-étape b-1).

Ainsi, un procédé préféré de préparation d'un composé de type dendrimère de génération (n) comprend les étapes suivantes :

a) une étape d'initiation comprenant une réaction d'allylation des groupes hydroxyles du glycérol ;
b) une étape optionnelle de propagation comprenant un cycle de deux étapes successives comprenant :

b-1) une étape de dihydroxylation du composé obtenu à l'étape a) ou à l'étape b-2),
b-2) et une étape d'allylation des groupes hydroxyles obtenus à l'étape b-1) ; et

c) une étape de terminaison comprenant une réaction de dihydroxylation des doubles liaisons carbone-carbone du composé obtenu à l'étape a) ou b-2) ;

dans lequel, l'étape optionnelle de propagation est mise en œuvre (n-1) fois, avec n compris entre 1 et 12.

Applications

La présente invention concerne l'utilisation d'au moins un composé de type dendrimère de génération (n) tel que défini dans la présente demande, pour le piégeage d'au moins un composé chimique, de préférence un polluant, contenu dans un échantillon liquide.

Par « au moins un composé de type dendrimère de génération (n) », on entend un composé de type dendrimère de génération (n) tel que défini dans la présente demande, ou un mélange de plusieurs composés de type dendrimère de génération (n) avec n représentant un nombre entier identique ou différent.

Par «piégeage », on entend la rétention d'au moins un composé chimique par au moins un composé de type dendrimère de génération (n). En particulier, le « piégeage » inclut notamment l'encapsulation et/ou la coordination dudit au moins un composé chimique par ledit au moins un composé de type dendrimère de génération (n). Le « piégeage » peut comprendre la formation d'interactions, telles que des liaisons hydrogènes, entre ledit au moins un composé chimique et ledit au moins un composé de type dendrimère de génération (n).

Selon un mode préféré de l'invention, le piégeage est réversible. Dans ce contexte, un « piégeage réversible » comprend en outre la libération dudit au moins un composé chimique piégé par ledit au moins un composé de type dendrimère de génération (n), notamment par action d'un réactif chimique, tel qu'un acide.

Par « composé chimique », on entend toute substance mono- ou polyatomique, organique ou inorganique, ionique ou neutre, pouvant être piégée par un composé de type dendrimère de génération (n) selon l'invention.

Selon un mode préféré de, le composé chimique est un polluant. Par « polluant », on entend toute substance qui développe un impact négatif sur tout ou partie d'un écosystème ou de l'environnement, par exemple, toute substance qui affecte la potabilité de l'eau.

Des exemples de polluants sont notamment des composés organiques d'origine naturelle ou synthétique incluant également leurs sels ou dérivés. Ces composés peuvent être notamment des composés biologiquement actifs, tels que ceux utilisés dans les domaines agricole, vétérinaire et de santé humaine. En particulier, ces composés peuvent être des pesticides, insecticides, herbicides, désherbants, des anti-inflammatoires, des antibiotiques, etc. Ces composés peuvent en outre être des perturbateurs endocriniens soupçonnés ou avérés. Des exemples de polluants sont notamment le 1,1,1-trichloroéthane, le 1,1-dichloroéthylène, le 1,2-dichloro-3-nitrobenzène, le 1,2-dichloroéthane, le dichlorométhane, le 1,2-dichlorobenzène, le 1-chloronaphthalène, le 2-chlorophénol, 2,4-dichlorophénol, 2,4,5-trichlorophénol, le diféconazole, l'époxiconazole, l'hexaconazole, le fluoranthène, l'isodrine, l'isoxaflutole, le benzène, le xylène, le toluène, le naphtalène, l'anthracène, l'atrazine, le methamidophos, le monolinuron, l'ométhoate, le phoxime, le glyphosate, le thiafluamide, la trifluraline, le 2,4,6-trichloroanisole (TCA), le diclofénac, le diuron, le 2,4,6-tribromoanisole (TBA) et certains perturbateurs endocriniens soupçonnés ou avérés, tels que le nonylphénol, le bis(2-éthylhexyl) phtalate, les bisphénols, le diéthylstilbestrol, l'estradiol ainsi que leurs sels ou leurs dérivés. De préférence, le polluant est le diclofénac, le glyphosate, l'atrazine, l'estradiol, le diuron et un de leurs sels ou dérivés.

Par « dérivé » on entend, un composé chimique ayant une structure chimique proche du composé chimique initial. Par exemple, ce composé « dérivé » peut avoir subi au moins une modification structurale par rapport au composé

initial. Selon un mode particulier, un composé « dérivé » est un sel tel que défini ci-dessus du composé initial. Particulièrement, un composé « dérivé » présente des propriétés similaires au composé chimique initial (non modifié), comme par exemple, un caractère polluant.

Le polluant peut également être un métal. Il est bien entendu que le terme « métal » inclut l'élément sous sa forme neutre mais aussi un cation ou un oxyde de l'élément. Particulièrement, il peut également inclure les composés organométalliques tels que le tributylétain.

Des exemples de métaux sont le mercure, le chrome, le cérium, le plomb, le cadmium, le cuivre, l'arsenic, le nickel, le zinc, le cobalt, le manganèse, l'antimoine, l'étain, et l'argent.

Selon un autre mode préféré de l'invention, le composé chimique peut également être un métal appartenant à la catégorie des terres rares. Des exemples de terres rares sont le scandium, l'yttrium ou un élément du groupe des lanthanides tel que le lanthane, le cérium ou le néodyme.

L'échantillon liquide contenant le composé chimique peut être un échantillon majoritairement constitué d'eau. Ledit échantillon liquide peut notamment provenir d'un effluent ménager, hospitalier, industriel ou minier, d'une station d'épuration, d'un cours d'eau et/ou d'une nappe phréatique. En variante, l'échantillon liquide peut être une solution du composé chimique dans un solvant organique tel que l'acétate d'éthyle, le THF, l'éthanol, le méthanol, l'acétone, le DMF, le DMSO, le dichlorométhane, le pentane et un mélange de ceux-ci.

L'invention concerne aussi un procédé de piégeage d'au moins un composé chimique contenu dans un échantillon liquide, comprenant les étapes suivantes :

(a) la mise en contact dudit échantillon avec au moins un composé de type dendrimère de génération (n) selon l'invention ;
(b) optionnellement, l'extraction dudit au moins un composé de type dendrimère de génération (n) piégeant ledit au moins un composé chimique, de préférence par dialyse ou ultrafiltration ;
(c) optionnellement, la libération dudit au moins un composé chimique par mise en contact d'un acide avec ledit au moins un composé de type dendrimère de génération (n) piégeant ledit au moins un composé chimique ; et
(d) optionnellement, la séparation dudit au moins un composé de type dendrimère de génération (n) et dudit au moins un composé chimique, de préférence par dialyse ou ultrafiltration.

Selon un mode préféré, le procédé de piégeage est réversible. Selon ce mode, les étapes (a) à (d) du procédé de piégeage tel que défini ci-dessus sont successivement mises en œuvre.

L'étape (a) du procédé de piégeage selon l'invention comprend la mise en contact de l'échantillon contenant au moins un composé chimique avec au moins un composé de type dendrimère de génération (n) de l'invention. Cette mise en contact peut être effectuée par mise en solution ou suspension, éventuellement sous agitation, dudit au moins un composé de type dendrimère de génération (n) dans l'échantillon liquide. En variante, ledit au moins un composé de type dendrimère de génération (n) peut être au préalable mis en solution ou suspension dans un liquide tel que l'eau, et la mise en contact peut résulter du mélange, de préférence sous agitation, de la solution ou suspension résultante et de l'échantillon liquide. Le liquide contenant ledit au moins un composé de type dendrimère de génération (n) et le liquide de l'échantillon liquide peuvent être identiques ou différents et peuvent être miscibles, partiellement miscibles ou non miscibles.

L'étape (a) de mise en contact permet le piégeage d'au moins un composé chimique contenu dans l'échantillon liquide par ledit au moins un composé de type dendrimère de génération (n). Il est bien entendu que la quantité dudit au moins un composé chimique piégé peut dépendre, par exemple, de la nature dudit au moins un composé chimique, dudit au moins un composé de type dendrimère de génération (n), et/ou des conditions opératoires, telles que la concentration.

L'étape (a) de mise en contact du procédé de piégeage selon l'invention est optionnellement suivie d'une étape (b) comprenant l'extraction dudit au moins un composé de type dendrimère de génération (n) piégeant ledit au moins un composé chimique. Cette extraction peut être réalisée par dialyse, par exemple à travers une membrane d'ester de cellulose, ou par ultrafiltration. En variante, l'extraction peut être réalisée au moyen d'une extraction liquide-liquide après décantation, en particulier lorsque le liquide contenant le composé de type dendrimère et le liquide de l'échantillon liquide ne sont pas miscibles.

Particulièrement, ledit au moins un composé de type dendrimère de génération (n) piégeant ledit au moins un composé chimique extrait à l'étape b) peut subir une étape de lyophilisation. Cette étape de lyophilisation peut notamment s'avérer utile pour déterminer la quantité dudit au moins un composé chimique piégé, par exemple par gravimétrie.

Ledit au moins un composé de type dendrimère de génération (n) piégeant ledit au moins un composé chimique extrait, lyophilisé ou non, peut être optionnellement remis en solution ou en suspension dans un liquide tel que l'eau en vue de libérer ledit au moins un composé chimique dudit au moins un composé de type dendrimère de génération (n) (étape c). Cette libération peut être mise en œuvre par mise en contact dudit au moins un composé de type dendrimère de génération (n) piégeant ledit au moins un composé chimique avec un acide.

Par « acide », on entend aussi bien les acides de Lewis que les acides de Brønsted. L'acide peut être un monoacide ou un polyacide. Des exemples d'acides sont notamment l'acide trifluoroacétique, l'acide acétique, l'acide propionique, l'acide chlorhydrique, l'acide bromhydrique, l'acide iodhydrique, l'acide fluorhydrique, l'acide sulfurique, l'acide phosphorique, l'acide nitrique, l'acide iodique, l'acide périodique, l'acide tétrafluoroborique, l'acide hexafluorophosphorique, les acides sulfoniques tels que l'acide méthanesulfonique, et les acides mono- et polycarboxyliques, de préférence l'acide chlorhydrique.

Ledit acide peut être utilisé pur, en suspension ou solution dans un solvant. Par solvant, on entend aussi bien un solvant organique tel que défini ci-dessus que l'eau. De préférence, ledit acide est utilisé en solution dans l'eau.

Une étape de séparation (étape d) dudit au moins un composé de type dendrimère de génération (n) et dudit au moins un composé chimique peut optionnellement être mise en œuvre. Par exemple, cette séparation peut être réalisée par simple filtration, par dialyse ou encore par ultrafiltration. Cette étape de séparation s'avère notamment nécessaire pour le recyclage dudit au moins un composé de type dendrimère de génération (n).

Selon un mode particulier de l'invention, ledit au moins un composé de type dendrimère de génération (n) obtenu à l'étape de séparation (étape d) du procédé tel que décrit ci-dessus est remis en œuvre à l'étape (a). Cette étape permet de recycler ledit au moins un composé de type dendrimère de génération (n) mis en œuvre dans le procédé de piégeage de l'invention. Selon ce mode particulier, on préfère que ledit au moins un composé de type dendrimère de génération (n) obtenu à l'étape (d) soit neutralisé par action d'une base telle que l'hydroxyde de sodium ou potassium, avant d'être remis en œuvre à l'étape (a). La quantité de base ajoutée peut être ajustée de sorte que le pH à l'issue de la neutralisation est compris entre 6,5 et 8. Par base, on entend notamment les bases de Brönsted et les bases de Lewis, telles que l'hydroxyde de sodium, l'hydroxyde de potassium, l'ammoniac, le carbonate de césium, le bicarbonate de sodium, l'hydrogénocarbonate de sodium, et le carbonate de potassium. De préférence, la base est l'hydroxyde de sodium.

De préférence, ledit au moins un composé de type dendrimère de génération (n) obtenu à l'étape (d) du procédé est remis en œuvre une ou plusieurs fois, typiquement au moins une fois, de préférence au moins trois fois, et de manière encore plus préférée huit fois.

Un mode de réalisation illustrant le procédé de piégeage réversible d'un polluant mettant en œuvre un composé de type dendrimère de génération (n) est notamment décrit par la Figure 2.

L'utilisation du composé de type dendrimère de génération (n) et le procédé de piégeage tels que décrits ci-dessus peuvent être mis en œuvre à grande échelle dans le cadre, par exemple, d'une exploitation industrielle.

Selon un mode particulier, l'invention concerne donc l'utilisation d'au moins un composé de type dendrimère de génération (n) ou la mise en œuvre d'un procédé de piégeage tels que décrits dans la présente demande pour la dépollution d'un effluent ménager, hospitalier, industriel ou minier.

Selon un autre mode particulier, l'invention concerne aussi l'utilisation d'au moins un composé de type dendrimère de génération (n) ou la mise en œuvre d'un procédé de piégeage tels que décrits dans la présente demande pour la dépollution d'un cours d'eau et/ou d'une nappe phréatique.

Selon un mode préféré, l'utilisation d'au moins un composé de type dendrimère de génération (n) ou la mise en œuvre d'un procédé de piégeage tels que définis dans la présente demande peuvent être mises au sein d'un module.

Un module peut être par exemple une station d'épuration dans laquelle est mise en œuvre le procédé de piégeage de l'invention telle qu'illustrée, par exemple dans la Figure 1 pour la dépollution d'un cours d'eau.

Un module peut également se présenter sous la forme d'un container pouvant contenir une arrivée de flux reliée à une enceinte de mise en contact avec le dendrimère, avec en sortie une unité d'ultrafiltration permettant de libérer le flux purifié. La solution résiduelle d'ultrafiltration est pompée dans un réacteur où un ajout d'acide tel que défini ci-dessus est réalisé. Ce mélange est de nouveau filtré (ultrafiltration), le rétentat est ensuite envoyé au niveau de la première enceinte avec un ajout immédiat de base telle que définie ci-dessus. Le filtrat récupéré contenant le polluant peut être ensuite concentré.

Selon un mode particulier de l'invention, le au moins un composé de type dendrimère de génération (n) et le procédé de piégeage mettant en œuvre ledit au moins un composé de type dendrimère de génération (n) peuvent être utilisés ou mis en œuvre pour l'extraction de composés chimiques, tels que des métaux, en particulier les terres rares.

## EXEMPLES

L'invention sera mieux comprise à la lumière des exemples suivants, qui sont donnés à titre purement illustratif et non limitatifs.

<u>**Exemple 1**</u> : Synthèse d'un composé de type dendrimère de génération (n)

### 1.1. Matériel et méthodes

<u>***Méthode 1** : Réaction d'allylation des fonctions hydroxyles*</u>

Dans un ballon bicol, la potasse broyée (1,2 éq. par fonction OH) a été mise sous vide pendant 10 min puis sous argon. Ensuite, 1 éq. du composé comprenant les fonctions hydroxyles (i.e. le glycérol ou le composé issu de la méthode 2), préalablement dissous dans du DMF, a été ajouté. Après 18h d'agitation à température ambiante, le bromure d'allyle (1,2 éq. par fonction OH) a été ajouté goutte à goutte à 0°C. Le mélange a été porté sous agitation pendant 48 h à 0°C, sous atmosphère inerte.

***Méthode de purification 1A*** **:**

Après 48 heures, de l'eau a été ajoutée pour neutraliser l'excès de KOH et la phase aqueuse a été extraite trois fois avec du dichlorométhane. Les phases organiques ont été rassemblées, séchées sur sulfate de magnésium et filtrées sur coton. Après évaporation sous pression réduite, le produit brut a été purifié par « flash » chromatographie sur gel de silice en utilisant un mélange Ether de pétrole (Essence G) / AcOEt (7:3) comme éluant. Le produit **II.1** a été obtenu sous la forme d'une huile jaune, avec un rendement de 65 %.

***Méthode de purification 1B*** **:**

Après 48 heures de réaction, de l'eau a été ajoutée et la phase aqueuse a été extraite trois fois avec du dichlorométhane. Les phases organiques ont été rassemblées, séchées sur sulfate de magnésium et filtrées sur coton. Après évaporation sous pression réduite, une précipitation a été effectuée (si nécessaire après vérification par RMN $^1$H) dans le dichlorométhane en utilisant un excès d'éther de pétrole. Le précipité a été séché sous vide à température ambiante.

<u>***Méthode 2** : Réaction de dihydroxylation des doubles liaisons carbone-carbone*</u>

L'AD-mix-β ((1,4 g pour 1 mmol de fonction allyle) x nombre de fonction allyle) a été ajouté à un mélange tBuOH / $H_2O$ (1:1). Le mélange résultant a été agité à température ambiante jusqu'à obtention de deux phases limpides puis refroidi à 0 °C. Après précipitation des sels dissous, le composé comprenant les fonctions allyles (i.e. le composé issu de la méthode 1) a été ajouté en une fois. Le mélange réactionnel a été maintenu à 0 °C pendant 48 heures. Le $Na_2SO_3$ (1,5 g pour 1,4 g d'AD-mix) a ensuite été ajouté à 0°C et le mélange réactionnel a été agité jusqu'à revenir à température ambiante. Le mélange réactionnel a été alors amené à sec sous pression réduite. Les impuretés ont été précipitées dans l'acétone. Après filtration, la phase organique a été évaporée sous pression réduite.

Le produit brut a été solubilisé dans de l'eau distillée puis une extraction à l'acétate d'éthyle a été effectuée. Le produit de dihydroxylation a été obtenu après évaporation sous pression réduite.

### 1.2. Synthèse d'un composé de type dendrimère de génération 1 (II.2)

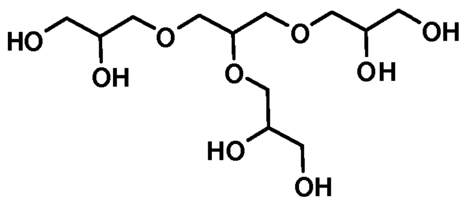

II.2

En utilisant le glycérol comme substrat initial, le composé de type dendrimère de génération 1 a été synthétisé par la mise en œuvre successive des méthodes suivantes : méthode 1 avec méthode de purification 1A (R = 65%), puis méthode 2 (R = 60%). Le composé de type dendrimère de génération 1 a été obtenu sous la forme d'une cire blanche.

- IR (film NaCl) = 1116 $cm^{-1}$ ($\upsilon_{CO\ ether}$) ; 2879-2931 $cm^{-1}$ ($\upsilon_{CH}$ et $\upsilon_{H2}$) ; 3423 $cm^{-1}$ ($\upsilon_{OH}$).
- RMN-$^1$H (500,13 MHz, $D_2O$, 298 K): δ (ppm) = 3,50-3,77 (16H, m) ; 3,81 (1H, m); 3,89 (3H, m).

- RMN-$^{13}C$ (125,76 MHz, $CDCl_3$, 298 K): $\delta$ (ppm) = 62,58 ; 70,30 ; 70,34-70,66 ; 70,81 ; 72,09 ; 77,86.
- SM-HR pour $C_{12}H_{26}O_9$ :

Masse calculée $[M+Na]^+$ = 337,1475 ;
Masse expérimentale $[M+Na]^+$ = 337,1483.

**1.3. Synthèse d'un composé de type dendrimère de génération 2 (II.4)**

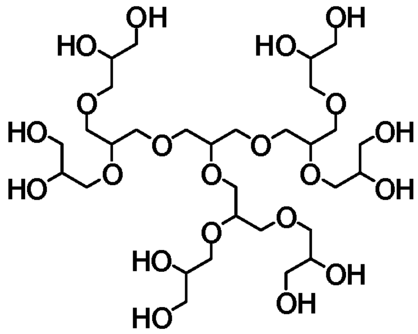

II.4

En utilisant le glycérol comme substrat initial, le composé de type dendrimère de génération 2 a été synthétisé par la mise en œuvre successive des méthodes suivantes : méthode 1 avec méthode de purification 1A (R = 65%), puis méthode 2 (R = 60%), puis méthode 1 avec méthode de purification 1B (R = 77%), puis méthode 2 (R = 69%). Le composé de type dendrimère de génération 2 a été obtenu sous la forme d'une cire blanche.

- IR (film NaCl) = 1116 $cm^{-1}$ ($\upsilon_{CO\ ether}$) ; 2852-2950 $cm^{-1}$ ($\upsilon_{CH}$ et $\upsilon_{H2}$) ; 3329 $cm^{-1}$ ($\upsilon_{OH}$).
- RMN-$^{1}H$ (600,16 MHz, $D_2O$, 298 K): $\delta$ (ppm) = 3,49-3,77 (40H, m) ; 3,79 (4H, m); 3,89 (6H, m).
- RMN-$^{13}C$ (150,91 MHz, $D_2O$, 298 K): $\delta$ (ppm) = 62,50-62,54 ; 70,28-70,40-70,81 ; 70,34-70,63 ; 72,07 ; 77,82-79,36.
- SM-HR pour $C_{30}H_{62}O_{21}$ :

Masse calculée $[M+Na]^+$ = 781,3681 ;
Masse expérimentale $[M+Na]^+$ = 781,3677.

**1.4. Synthèse d'un composé de type dendrimère de génération 3 (II.6)**

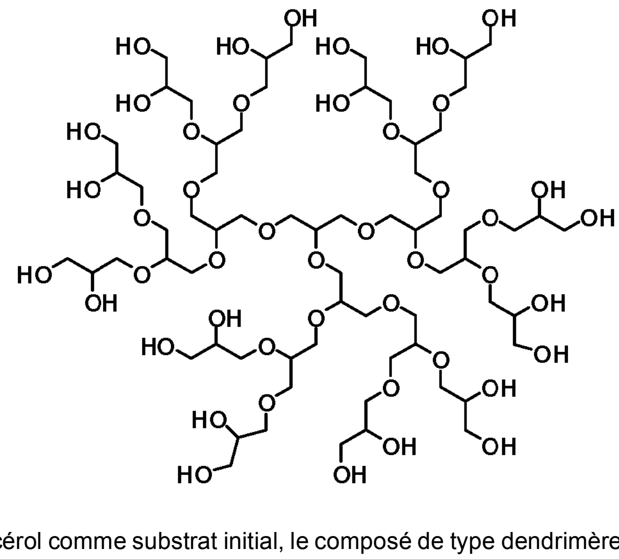

II.6

En utilisant le glycérol comme substrat initial, le composé de type dendrimère de génération 3 a été synthétisé par la mise en œuvre successive des méthodes suivantes : méthode 1 avec méthode de purification 1A (R = 65%), puis

méthode 2 (R = 60%), puis méthode 1 avec méthode de purification 1B (R = 77%), puis méthode 2 (R = 69%), puis méthode 1 avec méthode de purification 1B (R = 74%), puis méthode 2 (R = 71%). Le composé de type dendrimère de génération 3 a été obtenu sous la forme d'une cire blanche.

- IR (film NaCl) = 1121 $cm^{-1}$ ($\upsilon_{CO\ ether}$) ; 2863-2928 $cm^{-1}$ ($\upsilon_{CH}$ et $\upsilon_{H2}$) ; 3403 $cm^{-1}$ ($\upsilon_{OH}$).
- RMN-$^1$H (500,13 MHz, $D_2O$, 298 K): $\delta$ (ppm) = 3,49-3,76 (88H, m) ; 3,79 (10H, m); 3,89 (12H, m).
- RMN-$^{13}$C (150,91 MHz, $D_2O$, 298 K): $\delta$ (ppm) = 62,44-62,55 ; 70,28-70,42-70,82 ; 70,34-70,67 ; 72,09 ; 77,84-79,37.
- SM-HR pour $C_{66}H_{134}O_{45}$ :

Masse calculée $[M+Na]^+$ = 1670,7296 ;
Masse expérimentale $[M+Na]^+$ = 1670.

**Exemple 2** : Piégeage des métaux

**2.1. Matériel et méthodes**

Une solution aqueuse contenant l'ion métallique (6 mL, $C_f = 4.10^{-5}$ mol/L) a été mélangée avec une solution aqueuse de **II.6** (6 mL ; $2.10^{-6}$ mol/L) ; la solution résultante a été agitée pendant 2h à une vitesse d'agitation de 300 tours/min à température ambiante.

Une dialyse d'une heure a été réalisée en utilisant une membrane d'ester de cellulose (Cutoff 1000) dans de l'eau distillée (1L). Pour déterminer la quantité d'ion métallique extraite par le dendrimère, 10 mL de l'eau de dialyse ont été prélevés pour l'analyse ICP-AES (Spectrométrie à plasma à couplage inductif - Spectrométrie d'Emission Atomique).

L'analyse de l'eau de dialyse par ICP-AES (Thermo Scientific, iCAP 6000 Séries) a permis d'obtenir la quantité de métal résiduelle. La quantité d'ions ($M^{n+}$) présente dans la phase dendrimère a été obtenue par différence entre la quantité initiale et la quantité résiduelle.

Le pourcentage de rétention (% R) a été calculé selon l'expression suivante :

$$\% R = \frac{n\,(Maq\,(initial)) - n\,(Maq\,(\,final))}{n\,Maq\,(initial)} \times 100$$

avec n (Maq(initial)) et n (Maq(final)), les nombres de moles d'ions métalliques respectivement dans la solution de départ et dans l'eau de dialyse après extraction.

**2.2. Résultats**

Selon la procédure générale décrite ci-dessus, la capacité du dendrimère **II.6** à piéger les cations $Cu^{2+}$, $Ni^{2+}$, $Pb^{2+}$, $Cd^{2+}$, $Ag^+$, $Hg^{2+}$ et $Ce^{3+}$ a été étudiée. Les résultats sont illustrés dans le tableau 1 ci-dessous.

**Tableau 1 :**

| Cation métallique | | $Cu^{2+}$ | $Ni^{2+}$ | $Pb^{2+}$ | $Cd^{2+}$ | $Ag^+$ | $Hg^{2+}$ | $Ce^{3+}$ |
|---|---|---|---|---|---|---|---|---|
| Rapport = n (Maq (initial)) / n (II.6) | | 63,6 | 85,5 | 77 | 73 | 104,5 | 140 | 93 |
| % R | | 75 | 89 | 99 | 95,5 | 88 | 85 | >95 |
| Nombre d'équivalents piégés (éq.) | | 47,7 | 76 | 76 | 70 | 92 | 119 | >89 |
| **Test comparatif** | Rapport = n (Maq (initial)) / n (PPI-4) | 29,1 | | | | | 105,7 | |
| | **% R (PPI-4)** | 27 | | | | | 19 | |

Le composé de type dendrimère **II.6** présente une capacité de piégeage des cations métalliques supérieure à la capacité de piégeage du composé de type dendrimère PPI-4. En effet, la capacité de piégeage des cations métalliques $Cu^{2+}$ et $Hg^{2+}$ pour le composé II.6 est 3 à 4 fois plus élevée que celle du composé de type dendrimère PPI-4.

**Exemple 3** : Piégeage des composés organiques

### 3.1. Matériel et méthodes

*Méthode par dialyse*

Le dendrimère **II.6** (1 éq.) a été dissous dans l'eau (10 mL). Un excès de la molécule organique (100 éq.) a ensuite été ajouté. Le mélange a été agité pendant 18h à température ambiante. Une dialyse d'une heure a ensuite été effectuée en utilisant une membrane Cutoff 1000 dans de l'eau distillée (750 mL).

La quantité du polluant piégé a été déterminée par gravimétrie après lyophilisation.

*Extraction liquide-liquide*

Le procédé d'extraction liquide-liquide a été réalisé dans des piluliers agités avec une vitesse fixée à 300 tours/min à température ambiante. Une solution du dendrimère **II.6** (1 éq.) dans 7 mL d'eau a été mélangée avec un volume égal de solution de la molécule organique (100 éq.) dans l'acétate d'éthyle pendant 18 heures.

A la fin de l'extraction, les deux phases liquides ont été séparées par décantation. L'eau de la phase aqueuse a été éliminée par lyophilisation et la quantité du polluant piégé a été déterminée par gravimétrie.

### 3.2. Résultats

Selon la procédure générale décrite ci-dessus, la capacité du dendrimère **II.6** à piéger le β-estradiol (perturbateur endocrinien), le sel sodique du diclofénac (anti-inflammatoire), le glyphosate (herbicide), l'atrazine (herbicide) et le diuron (herbicide) a été étudiée.

Les résultats exposés dans le tableau 2 ci-dessous sont issus d'expériences réalisées avec 3 mg du dendrimère **II.6** et 100 équivalents de substrats.

Les tests comparatifs exposés dans ce tableau ont été réalisés avec 30 mg de dendrimère PPI-4, PAMAM-3, GD-PPI-4 ou GD-PAMAM-3, et 100 équivalents de substrats.

**Tableau 2** :

| *Substrats (100 éq.)* | **Diclofénac** | **Atrazine*** | **Diuron*** | **β-Estradiol*** | **Glyphosate** |
|---|---|---|---|---|---|
| ***II.6*** *Equivalents piégés (éq.) (=% piégeage)* | 37 | 40 | 39 | 30 | 41 |
| ***PPI-4*** *Equivalents piégés* | 65 | 77 | 79 | 84 | |
| ***PAMAM-3*** *Equivalents piégés* | 36 | 60 | 61 | 72 | |
| ***GD-PPI-4*** *Equivalents piégés* | 36 | 62 | 58 | 63 | |
| ***GD-PAMAM-3*** *Equivalents piégés* | 24 | 40 | 39 | 45 | |
| * Solvant organique utilisé : acétate d'éthyle (II.6) ou THF (PPI-4, PAMAM-3, GD-PPI-4, GD-PAMAM-3) | | | | | |

Ces résultats démontrent la capacité du dendrimère **II.6** à piéger les composés organiques testés.

**Exemple 4** : Méthode pour le recyclage d'un composé de type dendrimère de génération (n)

Le composé de type dendrimère de génération (n) comprenant le polluant a été dissous dans 10 mL d'eau distillée et une solution de HCl à 1 M a été ajoutée jusqu'à pH = 3. Le composé de type dendrimère de génération (n) sous sa forme protonnée a ensuite été purifié par microfiltration (filtre à seringue). Ensuite, un traitement basique a été effectué en ajoutant quelques gouttes de NaOH à 1 M jusqu'à revenir au pH initial des dendrimères (pH = 8-12).

### Revendications

1. Utilisation d'au moins un composé de type dendrimère de génération (n) ou un de ses sels comprenant :

   - un motif central de valence 3 représenté par la formule (I) :

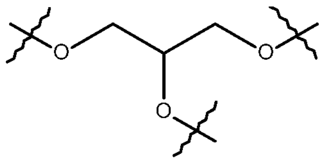

(I) ;

- (n-1) couches internes de génération composées d'unités de répétitions disposées en arborescence autour du motif central et représentées par la formule (II) :

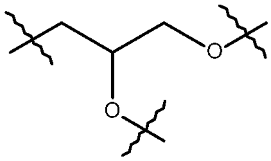

(II) ;

- une couche externe composée d'unités représentées par la formule (III) :

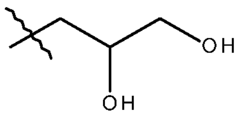

(III) ;

lesdites unités de formule (III) étant liées audit motif central quand n est 1 ou à la couche interne la plus éloignée dudit motif central quand n est différent de 1; et dans lequel n est un entier compris entre 1 et 12,
pour le piégeage d'au moins un composé chimique, de préférence un polluant, contenu dans un échantillon liquide.

2. Utilisation selon la revendication 1, dans laquelle ladite couche externe comprend $3 \times 2^{(n-1)}$ unités de formule (III).

3. Utilisation selon la revendication 1 ou 2, dans laquelle n est un entier compris entre 1 et 10, de préférence entre 1 et 5, et de manière encore plus préférée n est 1, 2, ou 3.

4. Utilisation selon l'une quelconque des revendications 1 à 3, dans laquelle ledit composé de type dendrimère de génération (n) est représenté par l'une des structures suivantes :

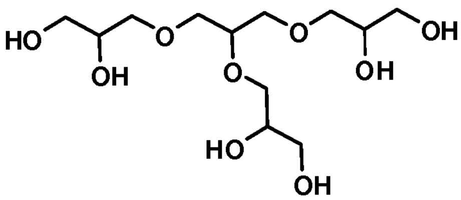

II.2,

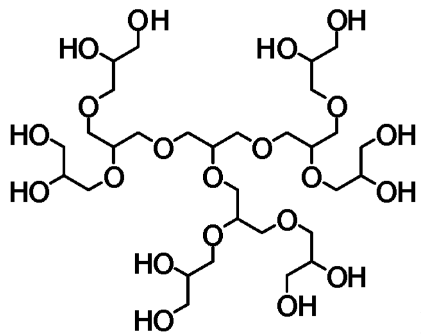

II.4, et

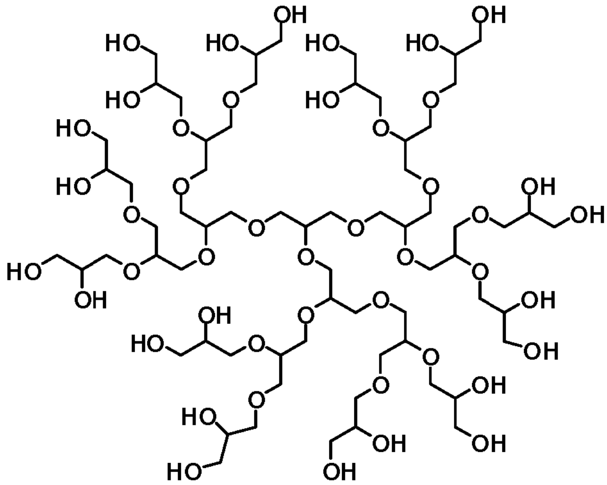

II.6.

5. Procédé de piégeage d'au moins un composé chimique, de préférence un polluant, contenu dans un échantillon liquide, comprenant les étapes suivantes :

(a) la mise en contact dudit échantillon avec au moins un composé de type dendrimère de génération (n) tel que défini selon l'une quelconque des revendications 1 à 4 ;
(b) optionnellement, l'extraction dudit au moins un composé de type dendrimère de génération (n) piégeant ledit au moins un composé chimique, de préférence par dialyse ou ultrafiltration ;
(c) optionnellement, la libération dudit au moins un composé chimique par mise en contact d'un acide ou d'un sel complexant avec ledit au moins un composé de type dendrimère de génération (n) piégeant ledit au moins un composé chimique ; et
(d) optionnellement, la séparation dudit au moins un composé de type dendrimère de génération (n) et dudit au moins un composé chimique, de préférence, par dialyse ou ultrafiltration.

6. Procédé selon la revendication 5, dans lequel ledit au moins un composé de type dendrimère de génération (n) obtenu à l'étape (d) est remis en œuvre à l'étape (a).

7. Utilisation selon l'une quelconque des revendications 1 à 4 ou procédé selon la revendication 5 ou 6, dans lequel ledit polluant est un métal, de préférence le mercure, le chrome, le cérium, le plomb, le cadmium, le cuivre, l'arsenic, le nickel, le zinc, le cobalt, le manganèse, l'antimoine, l'étain et l'argent.

8. Utilisation selon l'une quelconque des revendications 1 à 4 ou procédé selon la revendication 5 ou 6, dans lequel ledit polluant est un composé organique d'origine naturelle ou synthétique, de préférence le diclofénac, le glyphosate, l'atrazine, l'estradiol, le diuron, ou un de leurs sels et/ou un de leurs dérivés.

9. Utilisation selon l'une quelconque des revendications 1 à 4 pour la dépollution d'un effluent ménager, hospitalier, industriel, ou minier, et/ou pour la dépollution d'un cours d'eau et/ou d'une nappe phréatique, et/ou pour l'extraction de composés chimiques, tels que des métaux, en particulier les terres rares.

10. Composé de type dendrimère de génération (n) ou un de ses sels comprenant :

- un motif central de valence 3 représenté par la formule (I) :

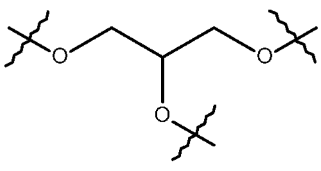

(I) ;

- (n-1) couches internes de génération composées d'unités de répétitions disposées en arborescence autour du motif central et représentées par la formule (II) :

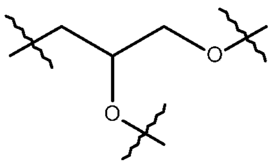

(II) ;

- une couche externe composée d'unités représentées par la formule (III) :

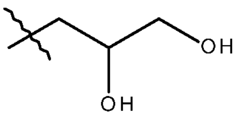

(III),

lesdites unités de formule (III) étant liées à la couche interne la plus périphérique (éloignée) dudit motif central ; et dans lequel n est un entier compris entre 3 et 12.

**11.** Composé de type dendrimère de génération (n), selon la revendication 10, représenté par la structure suivante :

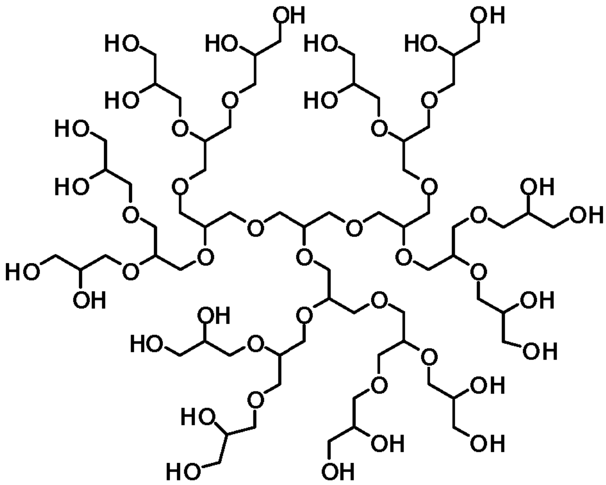

II.6.

**Patentansprüche**

**1.** Verwendung wenigstens einer Dendrimerverbindung der Generation (n) oder eines Salzes davon, umfassend:

- eine trivalente zentrale Kerneinheit, die mit der Formel (I) dargestellt ist:

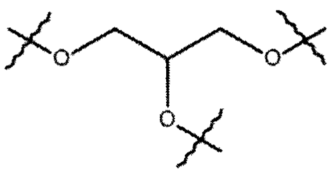

(I) ;

- (n-1) innere Generationsschichten, die aus Wiederholungseinheiten bestehen, die in einer baumartigen Struktur um die zentrale Kerneinheit angeordnet und mit der Formel (II) dargestellt sind:

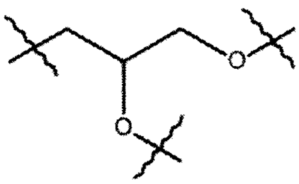

(II) ;

- eine äußere Schicht, die aus Einheiten besteht, die mit der Formel (III) dargestellt sind:

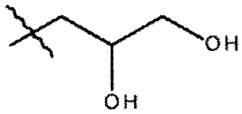

(III) ;

wobei die Einheiten der Formel (III) an die zentrale Kerneinheit gebunden sind, wenn n 1 ist, oder an die von der zentralen Kerneinheit entfernteste innere Schicht gebunden sind, wenn n von 1 verschieden ist; und wobei n eine ganze Zahl zwischen 1 und 12 ist,
zum Einfangen wenigstens einer chemischen Verbindung, vorzugsweise eines Schadstoffes, die in einer flüssigen Probe enthalten ist.

2. Verwendung gemäß Anspruch 1, wobei die äußere Schicht $3 \times 2^{(n-1)}$ Einheiten der Formel (III) umfasst.

3. Verwendung gemäß Anspruch 1 oder 2, wobei n eine ganze Zahl zwischen 1 und 10, vorzugsweise zwischen 1 und 5 ist und besonders bevorzugt n 1, 2 oder 3 ist.

4. Verwendung gemäß einem der Ansprüche 1 bis 3, wobei die Dendrimerverbindung der Generation (n) mit einer der folgenden Strukturen dargestellt ist:

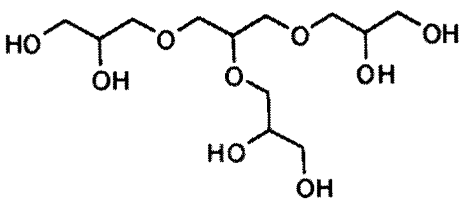

II.2,

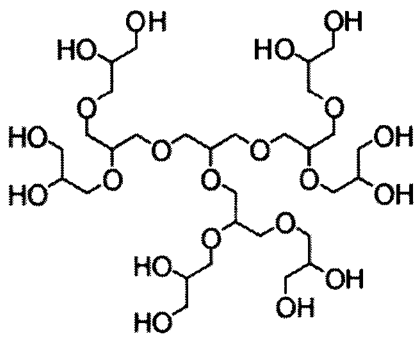

II.4 und

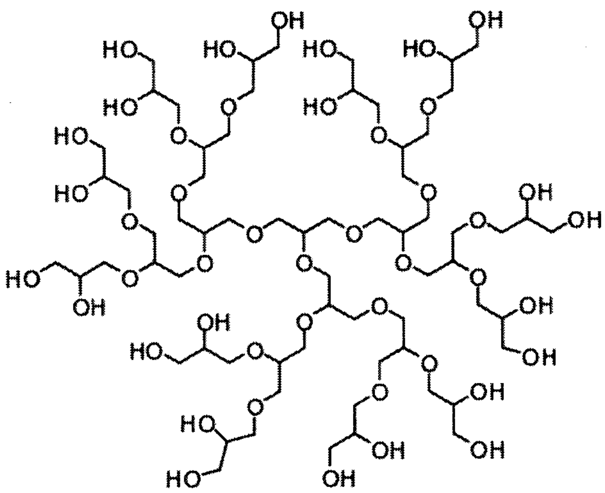

II.6.

5. Verfahren zum Einfangen wenigstens einer chemischen Verbindung, vorzugsweise eines Schadstoffes, die in einer flüssigen Probe enthalten ist, umfassend die folgenden Schritte:

(a) Inkontaktbringen der Probe mit wenigstens einer Dendrimerverbindung der Generation (n), wie gemäß einem der Ansprüche 1 bis 4 definiert;
(b) gegebenenfalls Extraktion der wenigstens einen Dendrimerverbindung der Generation (n), die die wenigstens eine chemische Verbindung einfängt, vorzugsweise mittels Dialyse oder Ultrafiltration;
(c) gegebenenfalls Freisetzung der wenigstens einen chemischen Verbindung durch Inkontaktbringen einer Säure oder eines Salzes zur Komplexbildung mit der wenigstens einen Dendrimerverbindung der Generation (n), die die wenigstens eine chemische Verbindung einfängt; und
(d) gegebenenfalls Trennung der wenigstens einen Dendrimerverbindung der Generation (n) und der wenigstens einen chemischen Verbindung vorzugsweise mittels Dialyse oder Ultrafiltration.

6. Verfahren gemäß Anspruch 5, wobei die in Schritt (d) erhaltene wenigstens eine Dendrimerverbindung der Generation (n) in Schritt (a) erneut eingesetzt wird.

7. Verwendung gemäß einem der Ansprüche 1 bis 4 oder Verfahren gemäß Anspruch 5 oder 6, wobei der Schadstoff ein Metall, vorzugsweise Quecksilber, Chrom, Cer, Blei, Cadmium, Kupfer, Arsen, Nickel, Zink, Cobalt, Mangan, Antimon, Zinn und Silber ist.

8. Verwendung gemäß einem der Ansprüche 1 bis 4 oder Verfahren gemäß Anspruch 5 oder 6, wobei der Schadstoff eine organische Verbindung natürlichen oder synthetischen Ursprungs, vorzugsweise Diclofenac, Glyphosat, Atrazin, Estradiol, Diuron oder ein Salz davon und/oder ein Derivat davon ist.

9. Verwendung gemäß einem der Ansprüche 1 bis 4 zur Reinigung von Haushalts-, Krankenhaus-, Industrie- oder Bergbauabwasser und/oder zur Reinigung eines Wasserlaufs und/oder von Grundwasser und/oder zur Extraktion chemischer Verbindungen, wie Metalle, insbesondere seltener Erden.

10. Dendrimerverbindung der Generation (n) oder ein Salz davon, umfassend:

- eine trivalente zentrale Kerneinheit, die mit der Formel (I) dargestellt ist:

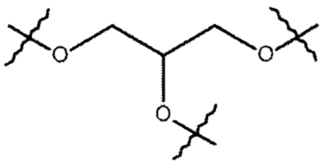

(I) ;

- (n-1) innere Generationsschichten, die aus Wiederholungseinheiten bestehen, die in einer baumartigen Struktur um die zentrale Kerneinheit angeordnet und mit der Formel (II) dargestellt sind:

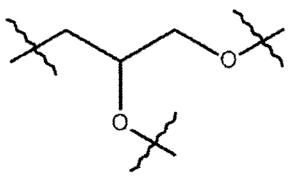

(II) ;

- eine äußere Schicht, die aus Einheiten besteht, die mit der Formel (III) dargestellt sind:

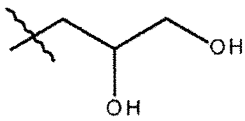

(III),

wobei die Einheiten der Formel (III) an die bezüglich der zentralen Kerneinheit äußerste (entfernteste) innere Schicht gebunden sind; und
wobei n eine ganze Zahl zwischen 3 und 12 ist.

**11.** Dendrimerverbindung der Generation (n) gemäß Anspruch 10, die mit der folgenden Struktur dargestellt ist:

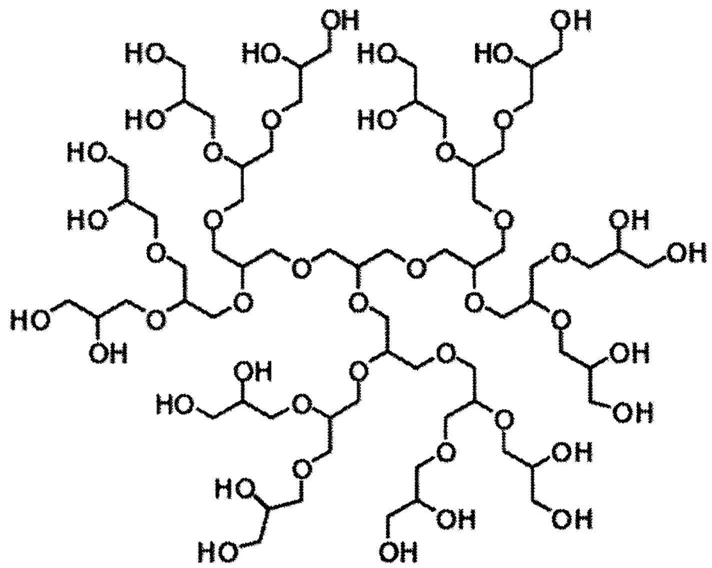

II.6.

## Claims

**1.** A use of at least one dendrimer-type compound of generation (n) or a salt thereof comprising:

- a central unit of valency 3 represented by the formula (I):

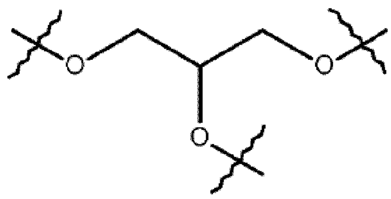

, (I);

- (n-1) inner generation layers consisting of repeat units arranged in a tree structure around the central unit and represented by the formula (II):

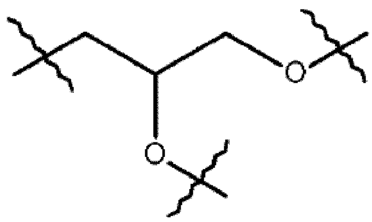

(II);

- an outer layer consisting of units represented by the formula (III):

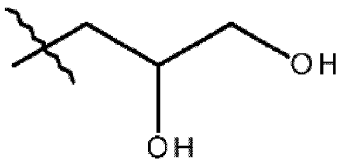

(III);

said units of formula (III) being bonded to said central unit when n is 1 or to the inner layer farthest from said central unit when n is different from 1; and where n is an integer comprised between 1 and 12, for trapping at least one chemical compound, preferably a pollutant, contained in a liquid sample.

**2.** The use according to claim 1, wherein said outer layer comprises $3 \times 2^{(n-1)}$ units of formula (III).

**3.** The use according to claim 1 or 2, wherein n is an integer comprised between 1 and 10, preferably between 1 and 5, and even more preferably n is 1, 2, or 3.

**4.** The use according to any one of claims 1 to 3, wherein said dendrimer-type compound of generation (n) is represented by one of the following structures:

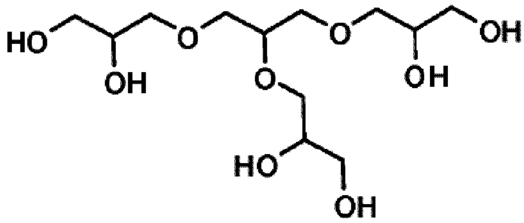

II.2,

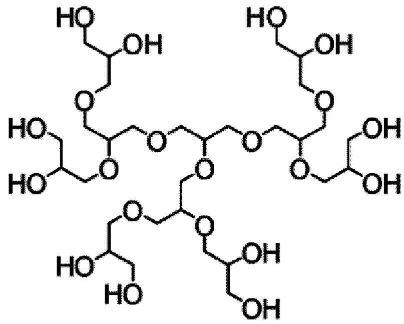

II.4, and

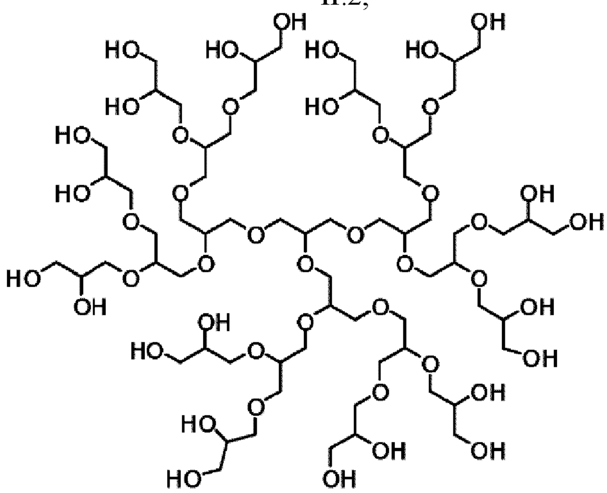

II.6.

**5.** A process for trapping at least one chemical compound, preferably a pollutant, contained in a liquid sample, comprising the following steps:

(a) contacting said sample with at least one dendrimer-type compound of generation (n) as defined according to any one of claims 1 to 4;
(b) optionally, extracting said at least one dendrimer-type compound of generation (n) trapping said at least one chemical compound, preferably by dialysis or ultrafiltration;
(c) optionally, releasing said at least one chemical compound by contacting an acid or a complexing salt with said at least one dendrimer-type compound of generation (n) trapping said at least one chemical compound; and
(d) optionally, separating said at least one dendrimer-type compound of generation (n) and said at least one

chemical compound, preferably, by dialysis or ultrafiltration.

**6.** The process according to claim 5, wherein said at least one dendrimer-type compound of generation (n) obtained in step (d) is used again in step (a).

**7.** The use according to any one of claims 1 to 4 or process according to claim 5 or 6, wherein said pollutant is a metal, preferably mercury, chromium, cerium, lead, cadmium, copper, arsenic, nickel, zinc, cobalt, manganese, antimony, tin and silver.

**8.** The use according to any one of claims 1 to 4 or process according to claim 5 or 6, wherein said pollutant is an organic compound of natural or synthetic origin, preferably diclofenac, glyphosate, atrazine, estradiol, diuron, or a salt thereof and/or a derivative thereof.

**9.** The use according to any one of claims 1 to 4 for depolluting a household, hospital, industrial, or mining effluent, and/or for depolluting a watercourse and/or a water table, and/or for extracting chemical compounds, such as metals, in particular rare earths.

**10.** A dendrimer-type compound of generation (n) or a salt thereof comprising:

- a central unit of valency 3 represented by the formula (I):

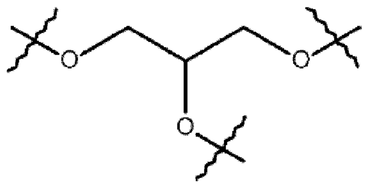

(I);

- (n-1) inner generation layers consisting of repeat units arranged in a tree structure around the central unit and represented by the formula (II):

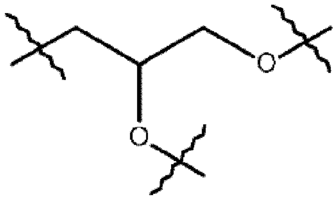

(II);

- an outer layer consisting of units represented by the formula (III):

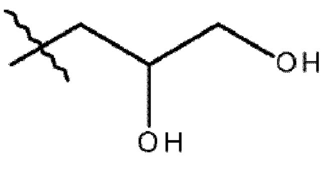

(III);

said units of formula (III) being bonded to the outermost (farthest) inner layer of said central unit; and where n is an integer comprised between 3 and 12.

**11.** The dendrimer-type compound of generation (n), according to claim 10, represented by the following structure:

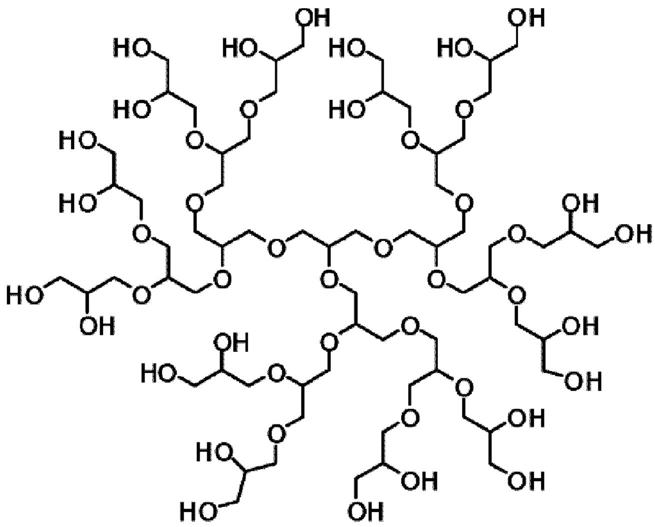

II.6

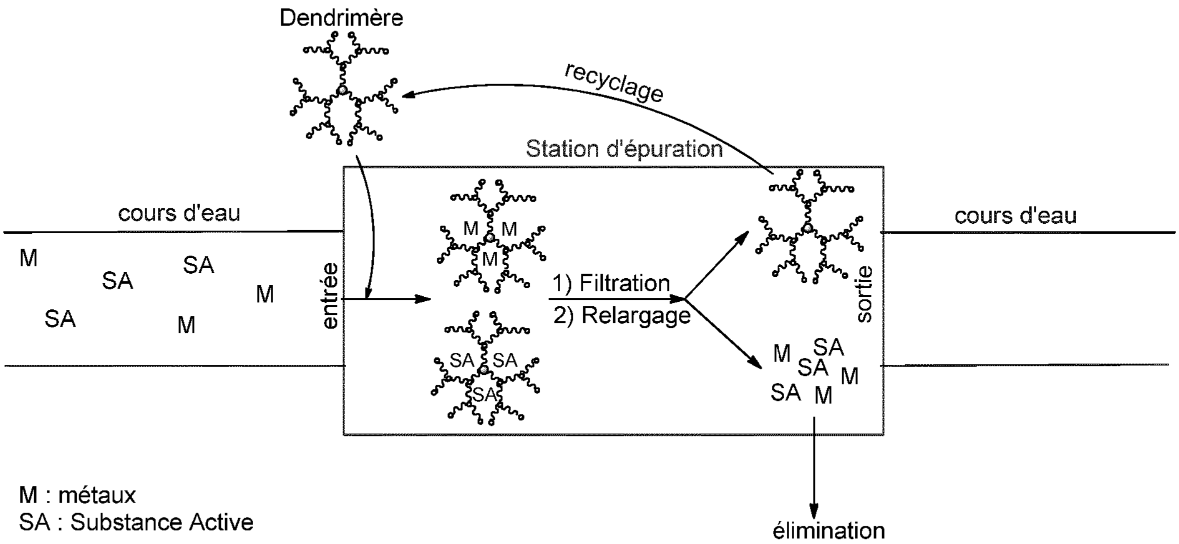


FIGURE 1

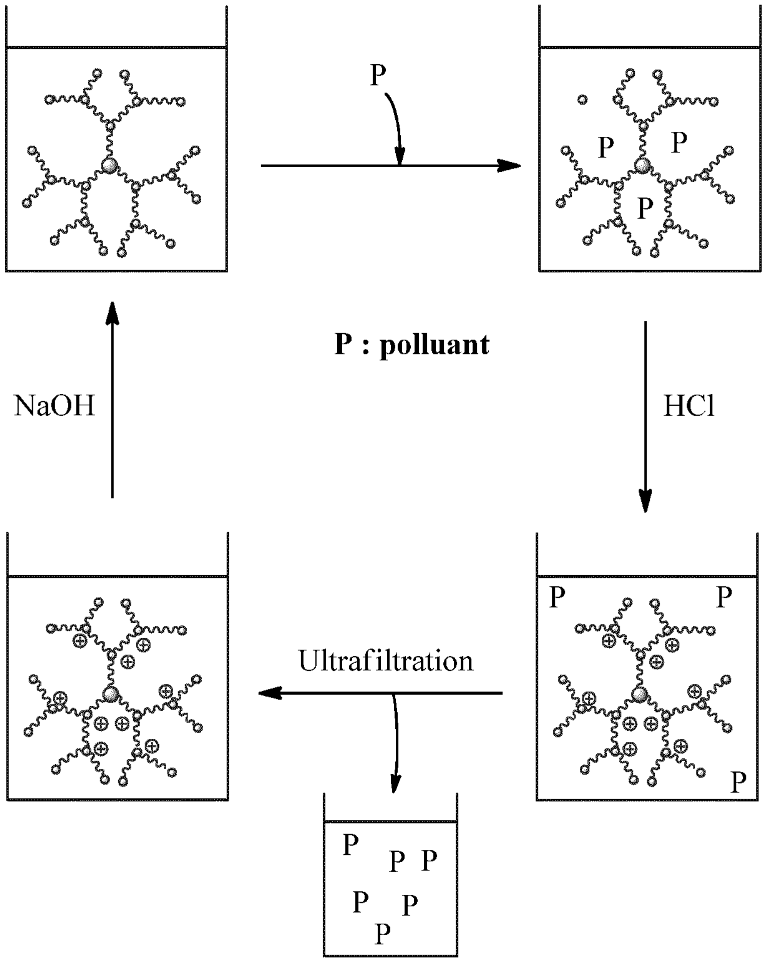


FIGURE 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- CA 2356474
- CN 102389746


### Littérature non-brevet citée dans la description


- **BALIEU et al.** *Adv. Synth. Catal.,* 2010, vol. 352, 1826-1833
- *J. Biomed. Mater. Res. A,* 2013, vol. 101A, 613-621
- **MENOT et al.** *Tetrahedron,* 2015, vol. 71, 3439-3446